# EUROPÄISCHE PATENTSCHRIFT

(11) **EP 1 398 304 B1**
(45) Veröffentlichungstag und Bekanntmachung des Hinweises auf die Patenterteilung: **27.08.2014**
(21) Anmeldenummer: 03019807.1
(22) Anmeldetag: 30.08.2003
(51) Int. Cl.: C07C 17/12, C07C 25/125

(54) **Verfahren zur Kernchlorierung von ortho-Xylol**
Process for the nuclear chlorination of ortho-xylene
Procédé de chloruration dans le noyau du xylène

(30) Priorität: 12.09.2002 DE 10242224
(43) Veröffentlichungstag der Anmeldung: 17.03.2004
(73) Patentinhaber: WeylChem Frankfurt GmbH, 65933 Frankfurt Griesheim (DE)
(72) Erfinder: Mack, Karl-Ernst, Dr., 65207 Wiesbaden (DE); Leitung, Hans-Jürgen, 65933 Frankfurt am Main (DE); Decker, Daniel, Dr., 65834 Liederbach a. Ts. (DE)
(74) Vertreter: Patentanwälte Zellentin & Partner

(56) Entgegenhaltungen:
- EP-A1- 0 173 222
- WO-A1-02/14245
- US-A- 4 190 609
- US-A- 4 289 916
- US-A- 4 444 983
- US-A- 4 647 709

## Beschreibung

Die vorliegende Erfindung betrifft ein Verfahren zur Kernchlorierung von ortho-Xylol zu einem Isomerengemisch von 4-Chlor-1,2-dimethylbenzol und 3-Chlor-1,2-dimethylbenzol in Gegenwart eines Katalysators und eines Co-Katalysators.

Monokernchlorierte ortho-Xylole sind wertvolle Zwischenprodukte zur Herstellung von Agro- und Pharmawirkstoffen.

Die Kernchlorierung von ortho-Xylol ist bekannt und erfolgt üblicherweise in Gegenwart von Friedel-Crafts-Katalysatoren wie z.B. Fe, FeCl₃, SbCl₃ oder SbCl₅. Man erhält auf diese Weise bei der Mono-Kernchlorierung ein Gemisch aus 4-Chlorund 3-Chlor-Isomeren im Verhältnis von geringer als 1.5 : 1 (US-A- 4,190,609). Da das 4-Chlor-lsomere das wertvollere der beiden ist, werden mehrere Verfahren zur Erhöhung seines Anteils beschrieben.

Durch Zusatz einfacher Schwefelverbindungen als Co-Katalysator kann der Anteil an 4-Chlor-1,2-dimethylbenzol z.B. durch Verwendung von Fe + S₂Cl₂ erhöht werden auf ein Verhältnis der 4-Chlor- zu 3-Chlor-lsomeren von 1,78 : 1 (Chemical Abstracts CA 1988, Nr. 472737).

Aus US-A-4,190,609 ist ein Verfahren zur Kernchlorierung von ortho-Xylol bekannt, wobei in Gegenwart von Lewis-Säuren als Katalysatoren und bestimmten substituierten Thianthrenen als Co-Katalysatoren gearbeitet wird. Dadurch erhöht sich zwar das Verhältnis der 4-Chlor- zu 3-Chlor-Isomeren auf bis zu 3,81 : 1, nachteilig jedoch bei der Verwendung der Thianthrene ist, dass dieses Verbindungen ähnlich wie Dioxine als hoch toxisch einzuschätzen sind.

In EP-A-126 669 wird die Kernchlorierung von ortho-Xylol mit SbCl₃ und N-Chlorcarbonyl-phenothiazin als Co-Katalysator beschrieben, wobei ein Verhältnis von 4-Chlor- zu 3-Chlor-Isomeren von 2,3 : 1 erreicht wird.

In WO 02/14245 werden Substituenten tragende benzokondensierte Thiazepine oder Thiazocine als Co-Katalysatoren zur Chlorierung von ortho-Xylol in Gegenwart von Lewis-Säuren beschrieben. Hochaktive Co-Katalysatoren dieser Verbindungsklassen, die zu Verhältnissen von 4-Chlor- zu 3-Chlor-Isomeren von grösser 3 : 1 führen, setzen jedoch aufwendig herzustellende Substitutionsmuster voraus.

Ein grundsätzlich anderes Verfahren zur Kernchlorierung von ortho-Xylol besteht in der Verwendung von heterogenen Katalysatoren wie z.B. Zeolithen. So wird in Chemical Abstracts CA 1991, Nr. 514135 durch Einsatz eines KL-Zeolithen in Nitrobenzol als Lösemittel von einem Verhältnis von 4-Chlor- zu 3-Chlor-Isomeren von 3.87: 1 berichtet. In J.Catal. 150, 1994, 430 - 433 wird unter Anwendung des speziellen Lösemittels 1,2-Dichlorethan zwar ein Isomerenverhältnis von 11.73 : 1 erreicht, der Umsatz an ortho-Xylol beträgt aber nur 60,6 %. Nachteilig beim Einsatz von Zeolithen ist die Notwendigkeit der Filtration bzw. wegen der Verwendung von Lösemitteln deren destillative Rückgewinnung.

In US 4,647,709 werden chlorierte Dimethylphenoxathiine als Co-Katalysatoren zum Einsatz bei Alkylbenzolen beschrieben, in den Beispielen wird ausschließlich Toluol verwendet. Die erzielten p/o-Verhältnisse sind mit 1,475 bis 1,67 nicht sehr hoch. Die US 4,289,916 betrifft die Chlorierung von Alkylbenzolen mit 2,3,7,8-substituierten Phenoxathiinen. Als Alkylbenzol wird wiederum nur Toluol genannt.

Die Aufgabe der vorliegenden Erfindung bestand darin, ein Verfahren zur Kernchlorierung von ortho-Xylol unter Verwendung eines einfach zu handhabenden Katalysatorsystems zur Verfügung zu stellen, wobei ein möglichst hohes Verhältnis von 4-Chlor- zu 3-Chlor-1,2-di-methylbenzol erreicht werden soll, das mindestens 3 : 1 beträgt.

Die gestellte Aufgabe wird auf überraschende Weise dadurch gelöst, dass als Co-Katalysator chlorsubstituiertes 2,8-Dimethylphenoxathiin eingesetzt wird.

Die Erfindung betrifft somit ein Verfahren zur Kernchlorierung von ortho-Xylol in Gegenwart eines Friedel-Crafts-Katalysators unter Zusatz von chloriertem, insbesondere tetrachloriertem 2,8-Dimethylphenoxathiin.

Als Chlorierungsmittel dient dabei beispielsweise Sulfurylchlorid oder elementares Chlor. Bevorzugtes Chlorierungsmittel ist elementares Chlor.

Geeignete Friedel-Crafts-Katalysatoren für des erfindungsgemäße Verfahren sind aus Lehrbüchern bekannt. Als Beispiele seien genannt: Antimonchloride, Aluminiumchlorid, Eisenchloride, Molybdänchloride, Titanchloride, Wolframchloride, Zinnchloride, Zinkchloride oder Bortrihalogenide, um nur einige zu nennen.

Es können auch Elemente oder Elementverbindungen, die während der Chlorierung einen Friedel-Crafts-Katalysator bilden, eingesetzt werden wie beispielsweise Oxide oder Salze, wie Carbonate oder Sulfide sowie auch andere Halogenide wie Fluoride, Bromide oder gegebenenfalls Jodide.

Bevorzugt werden im erfindungsgemäßen Verfahren Antimonchloride, Eisen, Eisenoxide, Eisensulfide und Eisen(lll)-chlorid eingesetzt. Besonders bevorzugt ist Eisen(III)-chlorid.
In einer ganz bevorzugten Ausführungsform wird Eisen z.B. in Form von Ringen eingesetzt, aus denen sich beim Durchströmen mit Chlor der aktive Friedel-Crafts-Katalysator bildet.

Friedel-Crafts-Katalysatoren und/oder deren Vorläufer können einzeln oder als beliebige Gemische untereinander eingesetzt werden, wobei die Menge bezogen auf das eingesetzte ortho-Xylol in weiten Grenzen von 0,0005 bis 5 Gew.-% variiert werden kann. Üblicherweise sind Mengen von 0,001 bis 1 Gew.-% ausreichend, bevorzugt werden 0,005 bis 0,5 Gew.-%. Beim Einsatz von Eisenringen wird z.B. ein Gehalt in der Reaktionslösung an gelöstem Eisen um 0,004 Gew.-% festgestellt.

Im erfindungsgemäßen Verfahren wird als Co-Katalysator vorzugsweise tetrachloriertes 2,8-Dimethyl-Phenoxathiin eingesetzt welches erhalten wird durch Chlorierung von 2,8-Dimethylphenoxatiin mit etwa 4 Mol Cl₂/Mol 2,8-Dimethylpheroxathiin in Gegenwart einer Lewis-Säure als Katalysator bei Temperaturen zwischen etwa 70°C und 120°C, gegebenenfalls in einen inerten Lösungsmittel. Seine Herstellung und Charakterisierung ist in EP-A-0 173 222 eingehend beschrieben. Dort wird ausgeführt, dass der Co-Katalysator hauptsächlich aus 1,3,7,9-Tetrachlor-2,8-Dimethyl-Phenoxathiin der Formel 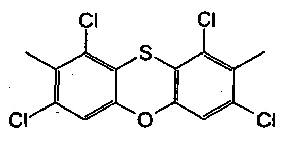 besteht.

Der erfindungsgemäße Co-Katalysator kann im weiten Mengenbereich von 0,001 bis 5 Gew.-% eingesetzt werden. Zweckmäßigerweise wird jedoch ein Mengenbereich von 0,001 bis 1 Gew.-% angewendet, bevorzugt werden 0,01 bis 0,5 Gew.-% bezogen auf das eingesetzte ortho-Xylol.

Das Gewichtsverhältnis von Friedel-Crafts-Katalysator bzw. Vorläufern davon zu dem Co-Katalysator kann im erfindungsgemäßen Verfahren in weiten Grenzen variiert werden. Geeignet ist z.B. ein Verhältnis von Friedel-Crafts-Katalysator bzw. Vorläufern davon zum Co-Katalysator von 500 : 1 bis 1 : 5, bevorzugt 10 : 1 bis 1 : 3, besonders bevorzugt 10 : 2,5 bis 1 : 2.

Das erfindungsgemäße Verfahren kann in Verdünnung mit Lösemittel durchgeführt werden. Geeignete Lösemittel sind solche, die unter den Bedingungen der Kernchlorierung nicht angegriffen werden wie z.B. Methylenchlorid, Chloroform, Tetrachlorkohlenstoff oder Essigsäure. Bevorzugt wird aber ohne Lösemittel gearbeitet.

Die Menge des eingesetzten Chlorierungsmittels kann so gewählt werden, dass ein Chlorierungsgrad von deutlich über 1 resultiert. Es ist dabei überraschend festzustellen, dass mit steigendem Chlorierungsgrad über 1 das Verhältnis von 4-Chlor-1,2-dimethylbenzol zu 3-Chlor-1,2-dimethylbenzol erheblich gesteigert werden kann. Beispielsweise wird bei der bevorzugten Chlorierung mit Chlor bei einem Chlorierungsgrad von 1,3 ein überraschend extrem hohes Verhältnis von 5,6 : 1 erreicht.

Die erfindungsgemäß durchgeführte Kernchlorierung kann grundsätzlich als Verfahren in der Flüssigphase im weiten Temperaturbereich des flüssigen Zustandes von ortho-Xylol und im Falle der Verwendung eines Lösemittels im Temperaturbereich des flüssigen Zustandes des Gemischs von ortho-Xylol und des Lösemittels erfolgen. Allgemein ist mit abnehmender Temperatur der Reaktion eine leichte Verbesserung des Verhältnisses des 4-Chlor- zu 3-Chlor-Isomeren festzustellen. Der Temperaturbereich für die Reaktion liegt allgemein günstig bei -20 bis 120°C, bevorzugt bei -5 bis 90°C, besonders bevorzugt bei 0 bis 60°C.

Der Reaktionsdruck kann normal, vermindert oder erhöht sein und ist grundsätzlich unkritisch. Bevorzugt wird aus apparatetechnischen Gründen bei Normaldruck gearbeitet.

Der Wassergehalt der Reaktionsmischung ist allgemein unkritisch und daher nicht der Einsatz speziell getrockneter Einsatzstoffe erforderlich. Es ist jedoch zweckmäßig z.B. aus Korrosionsgründen wegen des bei der Reaktion freiwerdenden Chlorwasserstoffs Wassergehalte im Reaktionsgemisch von weniger als 200 ppm anzustreben.

Für die Durchführung des erfindungsgemäßen Verfahrens ist die Reihenfolge der Zugabe der einzelnen Komponenten zum Reaktionsgemisch beliebig.
Das Verfahren kann sowohl diskontinuierlich als auch kontinuierlich durchgeführt werden. Bei der diskontinuierlichen Durchführung wird z.B. ortho-Xylol vorgelegt, Friedel-Crafts-Katalysator oder ein Vorläufer wie z.B. Eisenringe und Co-Katalysator hinzugegeben und nach Einstellen der gewünschten Reaktionstemperatur das Chlorierungsmittel, bevorzugt Chlor zudosiert. Bei der kontinuierlichen Fahrweise wird nach diskontinuierlicher Startreaktion gleichförmig und gleichzeitig ortho-Xylol, das beide Katalysatortypen gelöst enthält, und Chlor dosiert. Die kontinuierliche Durchführungsweise kann in den dafür allgemein bekannten Reaktoren wie Strömungsrohr, Umlaufreaktor, Kessel-Kaskade oder Blasensäule erfolgen. Bevorzugt wird bei der kontinuierlichen Umsetzung eine Blasensäule verwendet in der z.B. ein Bett aus Eisenringen vom Reaktionsgemisch durchströmt wird und dabei der Friedel-Crafts-Katalysator stets in situ gebildet wird.
Das Chloriergemisch kann direkt durch Destillation aufgearbeitet werden. Der im Sumpf verbleibende Co-Katalysator kann bei Bedarf daraus zurückgewonnen werden.

Das erfindungsgemäße Verfahren erlaubt die Kernchlorierung von ortho-Xylol mit hohen Anteilen an 4-Chlor-1,2-dimethylbenzol unter Aufwendung geringster Mengen an Friedel-Crafts- und Co-Katalysator sowie eine einfache Aufarbeitung des Reaktionsgemischs durch direkte Destillation. Das Verhältnis der 4-Chlor- zu 3-Chlor-lsomeren beträgt mindestens 3 : 1 und kann durch die Wahl des Chlorierungsgrads von über 1 wie z.B. beim Chlorierungsgrad 1,3 auf 5,6 : 1 gesteigert werden.

Die folgenden Beispiele verdeutlichen das erfindungsgemäße Verfahren, ohne es jedoch darauf zu beschränken.

### Beispiel 1

In ein Doppelmantelgefäß mit 15 cm Innendurchmesser und 90 cm Höhe werden 6784 g (64 mol) ortho-Xylol gegeben, 3,4 g Co-Katalysator darin aufgelöst und bei 20°C am Boden des Reaktors innerhalb von 5 Stunden 4544 g (64 mol) Chlor über eine Glasfritte eingeleitet. Um die Glasfritte herum befindet sich eine Schüttung aus Eisenringen. Nach kurzer Sättigungsphase des Reaktorinhalts mit Chlor beginnt die heftige Freisetzung von Chlorwasserstoff unter gleichzeitiger Erwärmung, die durch Kühlung auf 20°C abgefangen wird.
Nach beendeter Reaktion wird ein Gehalt an gelöstem Eisen von 35 ppm festgestellt. Die gaschromatografische Analyse des Reaktionsgemischs ergab 6,9 % ortho-Xylol, 65,6 % 4-Chlor-1,2-dimethylbenzol, 21,4 % 3-Chlor-1.2-dimethylbenzol, 5,5 % dichloriertes ortho-Xylol und 0,3 % Hochsieder. Das Verhältnis von 4-Chlor- zu 3-Chlor-Isomeren beträgt 3,06.

### Beispiel 2

In einem Glaskolben mit Rührer und einem Gas-Einleitrohr werden 2 mol ortho-Xylol mit 0,2 g FeCl₃ und 0,2 g Co-Katalysator versetzt, auf -5°C abgekühlt und unter Rühren innerhalb von 2,5 Stunden mit 2,25 mol Chlor (Chlorierungsgrad 1,125) begast. Die gaschromatographische Analyse zeigte vollständigen Umsatz von ortho-Xylol und ergab 70,1 % 4-Chlor-1,2-dimethylbenzol, 18,9 % 3-Chlor-1,2-dimethylbenzol und 11 % di- und höherchloriertes ortho-Xylol. Das Verhältnis von 4-Chlor- zu 3-Chlor-Isomeren beträgt 3,7.

### Beispiel 3

Analog zum Verfahren wie in Beispiel 2 wird die Reaktion bei 7°C durchgeführt und der Chlorierungsgrad 1,3 gewählt. Die gaschromatographische Analyse weist 61,2 % 4-Chlor-1,2-dimethylbenzol und 10,8 % 3-Chlor-1,2-dimethylbenzol aus und ergibt somit ein Verhältnis der 4-Chlor- zu 3-Chlor-lsomeren von 5,66.

## Patentansprüche

1. Verfahren zur Kernchlorierung von ortho-Xylol, **dadurch gekennzeichnet, dass** ortho-Xylol mit einem Chlorierungsmittel in Gegenwart mindestens eines Friedel-Crafts-Katalysators und eines tetrachlorierten 2,8-Dimethyl-Phenoxethiins als Co-Katalysator umgesetzt wird.

2. Verfahren gemäss Anspruch 1, **dadurch gekennzeichnet, dass** 1, 3, 7, 9-Tetrachlor-2, 8-dimethylphenoxathiin der Formel 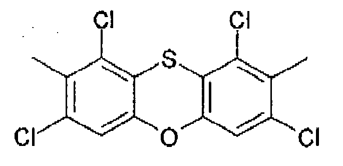 verwendet wird.

3. Verfahren nach Anspruch 1 oder 2, **dadurch gekennzeichnet, dass** elementares Chlor oder Sulfurylchlorid als Chlorierungsmittel, verwendet wird.

4. Verfahren nach mindestens einem der vorhergehenden Ansprüche, **dadurch gekennzeichnet, dass** der Co-Katalysator in einer Menge von 0,001 bis 5 Gew.-% bezogen auf die Menge des eingesetzten ortho-Xylols verwendet wird.

5. Verfahren nach mindestens einem der vorhergehenden Ansprüche **dadurch gekennzeichnet, dass** das Verhältnis von Friedel-Crafts-Katalysator oder dessen Vorläufer in Form eines Elementes oder einer Elementverbindung, die während der Chlorierung den Friedel-Crafts-Katalysator bildet, zum Co-Katalysator im Bereich von 500:1 bis 1:5 liegt.

6. Verfahren nach mindestens einem der vorhergehenden Ansprüche **dadurch gekennzeichnet, dass** das Verfahren ohne Zusatz eines Lösungsmittels durchgeführt wird.

7. Verfahren nach mindestens einem der vorhergehenden Ansprüche **dadurch gekennzeichnet, dass** das Verfahren bei einer Temperatur im Bereich von -20 bis +120 °C durchgeführt wird.

## Claims

1. Method for ring chlorinating ortho-xylene, **characterized in that** the ortho-xylene is reacted with a chlorinating agent in the presence of at least one Friedel-Crafts catalyst and a tetra-chlorinated 2,8-dimethyl-phenoxathiin as co-catalyst.

2. Method according to claim 1, **characterized in that** 1, 3, 7, 9-tetra-chloro-2.8-dimethyl-phenoxathiin of the formula 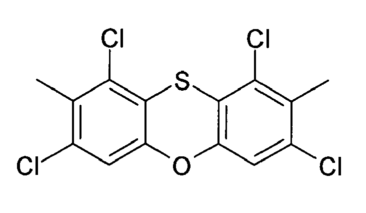 is used.

3. Method according to claim 1 or 2, **characterized in that** elemental chlorine or sulphuryl chloride is used as chlorinating agent.

4. Method according to at least one of the preceding claims, **characterized in that** the co-catalyst is used an amount of 0.001 to 5 weight -% based on the amount of ortho-xylene used.

5. Method according to at least one of the preceding claims, **characterized in that** the ratio of Friedel-Crafts catalyst or precursors thereof, in the form of an element or element compound that generates the Friedel-Crafts catalyst during chlorination, to co-catalyst is in the range from 500:1 to 1:5.

6. Method according to at least one of the preceding claims, **characterized in that** the method is carried out without addition of a solvent.

7. Method according to at least one of the preceding claims, **characterized in that** the method is carried out at a temperature in the range from -20 to +120 °C.

## Revendications

1. Procédé pour la chloration du noyau d'ortho-xylène, **caractérisé en ce que** l'ortho-xylène est transformé avec un agent de chloration en présence d'au moins un catalyseur de Friedel-Crafts et d'une 2,8-diméthyl-phénoxathiine tétrachlorée comme cocatalyseur.

2. Procédé selon la revendication 1, **caractérisé en ce qu'**on utilise de la 1,3,7,9-tétrachloro-2,8-diméthylphénoxathiine de formule.

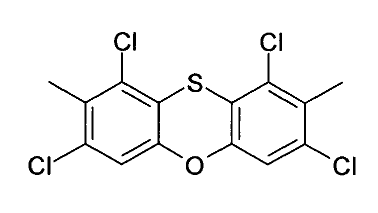

3. Procédé selon la revendication 1 ou 2, **caractérisé en ce qu'**on utilise du chlore élémentaire ou du chlorure de sulfuryle comme agent de chloration.

4. Procédé selon au moins l'une quelconque des revendications précédentes, **caractérisé en ce que** le cocatalyseur est utilisé en une quantité de 0,001 à 5% en poids par rapport à la quantité de l'ortho-xylène utilisée.

5. Procédé selon au moins l'une quelconque des revendications précédentes, **caractérisé en ce que** le rapport du catalyseur de Friedel-Crafts ou de son précurseur, sous forme d'un élément ou d'un composé élémentaire, qui forme le catalyseur de Friedel-Crafts pendant la chloration, au cocatalyseur se situe dans la plage de 500:1 à 1:5.

6. Procédé selon au moins l'une quelconque des revendications précédentes, **caractérisé en ce que** le procédé est réalisé sans addition d'un solvant.

7. Procédé selon au moins l'une quelconque des revendications précédentes, **caractérisé en ce que** le procédé est réalisé à une température dans la plage de -20 à +120°C.
